# EUROPÄISCHE PATENTSCHRIFT

(11) **EP 2 765 975 B1**
(45) Veröffentlichungstag und Bekanntmachung des Hinweises auf die Patenterteilung: **16.03.2016**
(21) Anmeldenummer: 12770151.4
(22) Anmeldetag: 11.10.2012
(51) Int. Cl.: A61K 6/02, C03C 3/095, C03C 3/097, C03C 4/00, C03C 10/00

(54) **LITHIUMSILIKAT-GLASKERAMIK UND -GLAS MIT DREIWERTIGEM METALLOXID**
LITHIUM SILICATE GLASS CERAMIC AND LITHIUM SILICATE GLASS COMPRISING A TRIVALENT METAL OXIDE
VITROCÉRAMIQUE ET VERRE EN SILICATE DE LITHIUM, AYANT UN OXYDE MÉTALLIQUE TRIVALENT

(30) Priorität: 14.10.2011 EP 11185336
(43) Veröffentlichungstag der Anmeldung: 20.08.2014
(73) Patentinhaber: Ivoclar Vivadent AG, 9494 Schaan (LI)
(72) Erfinder: RITZBERGER, Christian, CH-9472 Grabs (CH); APEL, Elke, CH-9479 Oberschan (CH); HÖLAND, Wolfram, FL-9494 Schaan (LI); RHEINBERGER, Volker, FL-9490 Vaduz (LI)
(74) Vertreter: UEXKÜLL & STOLBERG
(86) Internationale Anmeldenummer: PCT/EP2012/070221
(87) Internationale Veröffentlichungsnummer: WO 2013/053865

(56) Entgegenhaltungen:
- EP-A2- 1 577 275
- US-A- 3 463 647
- US-A1- 2002 009 600
- US-A1- 2006 040 142
- US-A1- 2007 042 889
- US-B1- 6 458 728
- US-B1- 6 514 890
- US-B1- 6 524 982
- JAKOVAC M ET AL: "Measurement of ion elution from dental ceramics", JOURNAL OF THE EUROPEAN CERAMIC SOCIETY, ELSEVIER SCIENCE PUBLISHERS, BARKING, ESSEX, GB, Bd. 26, Nr. 9, 1. Januar 2006 (2006-01-01) , Seiten 1695-1700, XP024960679, ISSN: 0955-2219, DOI: 10.1016/J.JEURCERAMSOC.2005.03.242 [gefunden am 2006-01-01]

## Beschreibung

Die Erfindung betrifft die verwendung von einer Lithiumsilikat-Glaskeramik und -Glas, die dreiwertiges Metalloxid ausgewählt aus Y₂O₃, La₂O₃, Yb₂O₃, Bi₂O₃ und Mischungen davon enthalten als Dentalmaterial, bevorzugt zur Herstellung von dentalen Restaurationen, eignen.

Lithiumsilikat-Glaskeramiken zeichnen sich in der Regel durch sehr gute mechanische Eigenschaften aus, weshalb sie seit langem im Dentalbereich und dort vornehmlich zur Herstellung von Dentalkronen und kleinen Brücken Anwendung finden. Die bekannten Lithiumsilikat-Glaskeramiken enthalten üblicherweise als Hauptkomponenten SiO₂, Li₂O, Na₂O oder K₂O, und Keimbildner wie P₂O₅.

Die DE 24 51 121 beschreibt Lithiumdisilikat-Glaskeramiken, die K₂O und Al₂O₃ enthalten. Sie werden aus entsprechenden keimhaltigen Ausgangsgläsern hergestellt, die zur Kristallisation von Lithiumdisilikat auf Temperaturen von 850 bis 870°C erwärmt werden.

Die EP 827 941 beschreibt sinterbare Lithiumdisilikat-Glaskeramiken für Dentalzwecke, die neben La₂O₃ auch K₂O oder Na₂O aufweisen. Die Erzeugung der Lithiumdisilikat-Kristallphase-erfolgt bei einer Temperatur von 850°C.

Aus der EP 916 625 sind Lithiumdisilikat-Glaskeramiken bekannt, die ebenfalls La₂O₃ und K₂O enthalten. Für die Bildung von Lithiumdisilikat wird eine Wärmebehandlung bei 870°C durchgeführt.

Die EP 1 505 041 beschreibt Lithiumsilikat-Glaskeramiken mit Gehalt an K₂O, die sich bei Vorliegen von Lithiummetasilikat als Hauptkristallphase sehr gut mechanisch z.B. mittels CAD/CAM-Verfahren bearbeiten lassen, um dann durch weitere Wärmebehandlung bei Temperaturen von 830 bis 850°C in hochfeste Lithiumdisilikat-Glaskeramiken überzugehen.

Die EP 1 688 398 beschreibt ähnliche K₂O- und Al₂O₃-haltige Lithiumsilikat-Glaskeramiken, die zudem im Wesentlichen frei von ZnO sind. Zur Erzeugung von Lithiumdisilikat wird bei ihnen eine Wärmebehandlung bei 830 bis 880° C angewandt.

Die US 5,507,981 beschreibt Verfahren zur Erzeugung von Dentalrestaurationen und in diesen Verfahren einsetzbare Glaskeramiken. Dabei handelt es sich insbesondere um Lithiumdisilikat-Glaskeramiken, die regelmäßig entweder Na₂O oder K₂O enthalten.

Die US 6,455,451 betrifft Lithiumdisilikat-Glaskeramiken, die auch K₂O enthalten. Die Erzeugung der gewünschten Lithiumdisilikat-Kristallphase erfordert allerdings hohe Temperaturen von 800 bis 1000°C.

Die WO 2008/106958 offenbart Lithiumdisilikat-Glaskeramiken zum Verblenden von Zirkonoxid-Keramiken. Die Glaskeramiken enthalten Na₂O und werden durch Wärmebehandlung von keimhaltigen Gläsern bei 800 bis 940°C erzeugt.

Die WO 2009/126317 beschreibt GeO₂-haltige Lithiummetasilikat-Glaskeramiken, die zudem K₂O aufweisen. Die Glaskeramiken werden vor allem durch maschinelle Bearbeitung zu Dentalprodukten verarbeitet.

Die WO 2011/076422 betrifft Lithiumdisilikat-Glaskeramiken, die neben hohen Gehalten an ZrO₂ oder HfO₂ auch K₂O aufweisen. Die Kristallisation von Lithiumdisilikat erfolgt bei Temperaturen von 800 bis 1040°C.

Den bekannten Lithiumdisilikat-Glaskeramiken ist gemeinsam, dass bei ihnen Wärmebehandlungen bei mehr als 800°C erforderlich sind, um die Ausscheidung von Lithiumdisilikat als Hauptkristallphase zu bewirken. Daher ist auch eine hohe Energiemenge zu ihrer Herstellung nötig. Weiter sind bei den bekannten Glaskeramiken regelmäßig die Alkalimetalloxide K₂O oder Na₂O sowie ZrO₂ als essentielle Komponenten vorhanden, die zur Erzeugung der Glaskeramiken mit den gewünschten Eigenschaften und insbesondere Bildung der angestrebten Lithiumdisilikat-Hauptkristallphase offenbar erforderlich sind.

Es besteht daher ein Bedarf an Lithiumsilikat-Glaskeramiken, bei deren Herstellung die Kristallisation von Lithiumdisilikat bei niedrigeren Temperaturen hervorgerufen werden kann. Weiter sollen sie auch ohne die bisher als erforderlich angesehenen Alkalimetalloxide K₂O oder Na₂O sowie von ZrO₂ herstellbar sein und aufgrund vor allem ihrer optischen und mechanischen Eigenschaften sich insbesondere zur Herstellung von dentalen Restaurationen eignen.

Diese Aufgabe wird durch die Verwendung von Lithiumsilikat-Glaskeramik nach einem der Ansprüche 1 bis 12 oder 15 bis 17 gelöst. Gegenstand der Erfindung sind ebenfalls die Verwendung von Ausgangsglas nach Anspruch 13 oder 15 bis 17, die Verwendung von Lithiumsilikatglas mit Keimen nach Anspruch 14 bis 17 sowie das Verfahren zur Herstellung einer Lithiumsilikat-Glaskeramik nach Anspruch 18.

Die erfindungsgemäß als Dentalmaterial verwendete Lithiumsilikat-Glaskeramik zeichnet sich dadurch aus, dass sie dreiwertiges Metalloxid ausgewählt aus Y₂O₃, La₂O₃, Yb₂O₃, Bi₂O₃ und Mischungen davon enthält, wobei die Glaskeramik im Wesentlichen frei von K₂O und Na₂O ist.

Bevorzugt ist das dreiwertige Metalloxid ausgewählt aus Y₂O₃, Yb₂O₃, Bi₂O₃ und Mischungen davon.

Es ist bevorzugt, dass die Glaskeramik das dreiwertige Metalloxid oder Mischungen davon in einer Menge von 0,1 bis 15, insbesondere 2,0 bis 10,0, besonders bevorzugt 2,0 bis 8,0 und besonders bevorzugt 2,4 bis 6,0 Gew.-% enthält.

Es ist besonders überraschend, dass die Bildung der erfindungsgemäßen Glaskeramik mit Lithiumdisilikat als Hauptkristallphase auch bei Abwesenheit verschiedener bei konventionellen Glaskeramiken als erforderlich angesehener Komponenten, wie insbesondere K₂O und Na₂O gelingt und dies sogar bei sehr niedrigen und damit vorteilhaften Kristallisationstemperaturen von insbesondere 630 bis 720°C. Auch besitzt die Glaskeramik eine Kombination von optischen und mechanischen Eigenschaften sowie Verarbeitungseigenschaften, die für den Einsatz als Dentalmaterial sehr vorteilhaft sind.

Weiter ist eine Glaskeramik bevorzugt, die weniger als 0,1 Gew.-% MgO und ZnO enthält. Ganz besonders bevorzugt ist die Glaskeramik im Wesentlichen frei von MgO und ZnO.

Auch ist eine Glaskeramik bevorzugt, bei der Lithiumsilikat-Glaskeramik ausgenommen ist, die mindestens 6,1 Gew.-% ZrO₂ enthält.

Weiter ist auch eine Glaskeramik bevorzugt, bei der Lithiumsilikat-Glaskeramik ausgenommen ist, die mindestens 8,5 Gew.-% Übergangsmetalloxid ausgewählt aus der Gruppe bestehend aus Oxiden von Yttrium, Oxiden von Übergangsmetallen mit der Ordnungszahl 41-79 und Mischungen dieser Oxide enthält.

Die erfindungsgemäße Glaskeramik enthält vorzugsweise 55,0 bis 85,0, insbesondere 60,0 bis 80,0 und bevorzugt 66,0 bis 78,0 Gew.-% SiO₂.

Auch ist es bevorzugt, dass die Glaskeramik 13,0 bis 21,0 und insbesondere 13,0 bis 20,0 und besonders bevorzugt 15,0 bis 18,0 Gew.-% Li₂O enthält.

Weiter ist es bevorzugt, dass das Molverhältnis zwischen SiO₂ und Li₂O zwischen 1,7 bis 3,1 und insbesondere 1,8 bis 3,0 liegt. Es ist sehr überraschend, dass innerhalb dieses breiten Bereichs die Erzeugung von Lithiumdisilikat gelingt. Gerade bei Verhältnissen von weniger als 2,0 bilden herkömmliche Materialien Lithiummetasilikat statt Lithiumdisilikat.

In einer weiteren bevorzugten Ausführungsform beträgt das Molverhältnis zwischen SiO₂ und Li₂O mindestens 2,2, insbesondere 2,3 bis 2,5, und bevorzugt etwa 2,4, da damit eine Glaskeramik mit besonders hoher Festigkeit erhalten wird.

Die erfindungsgemäße Glaskeramik kann auch Keimbildner enthalten. Besonders bevorzugt wird hierfür P₂O₅ verwendet. Vorzugsweise enthält die Glaskeramik 0 bis 8,0, insbesondere 2,0 bis 8,0, bevorzugt 2,5 bis 6,0 und ganz besonders bevorzugt 3,0 bis 5,0 Gew.-% P₂O₅.

In einer weiteren bevorzugten Ausführungsform enthält die Glaskeramik mindestens eine und bevorzugt alle folgenden Komponenten:

| Komponente | Gew.-% | | | | | | |
|---|---|---|---|---|---|---|---|
| SiO₂ | 68,0 | bis | 78,0 | | | | |
| Li₂O | 13,0 | bis | 20,0 | | | | |
| dreiwertiges Metalloxid | | | | | | | |
| und Mischungen davon | 2,0 | bis | 6,0 | | | | |
| Al₂O₃ | 0 | bis | 6,0 | | | | |
| Rb₂O und/oder Cs₂O | 0 | bis | 5,0 | | | | |
| P₂O₅ | 0 | bis | 4,5, | bevorzugt | 3,0 | bis | 4,5. |

Die erfindungsgemäße Glaskeramik kann darüber hinaus noch Zusatzkomponenten enthalten, die insbesondere ausgewählt sind aus Oxiden zweiwertiger Elemente, weiteren Oxiden vierwertiger Elemente, weiteren Oxiden fünfwertiger Elemente, Oxiden sechswertiger Elemente, Schmelzbeschleunigern, Färbemitteln und Fluoreszenzmitteln. Allerdings ist es dabei bevorzugt, dass die Glaskeramik frei von Arsenoxid und Antimonoxid ist. Diese Oxide werden bei Glaskeramiken für technische Anwendungen als Mittel zur Homogenisierung der Schmelze eingesetzt. Angesichts ihres gesundheitsschädigenden Potentials sind sie in der erfindungsgemäßen Glaskeramik zu vermeiden, da diese insbesondere als Dentalmaterial verwendet wird.

Als Oxide zweiwertiger Elemente kommen insbesondere die Erdalkalimetalloxide, vorzugsweise CaO, BaO, MgO, SrO oder eine Mischung davon und bevorzugt MgO und/oder CaO in Frage. Sie können in Mengen von 0 bis 5,0 Gew.-% verwendet werden.

Der Begriff "weitere Oxide vierwertiger Elemente" bezeichnet Oxide vierwertiger Elemente mit Ausnahme von SiO₂. Beispiele für geeignete weitere Oxide vierwertiger Elemente sind TiO₂, SnO₂ und GeO₂.

Der Begriff "weitere Oxide fünfwertiger Elemente" bezeichnet Oxide fünfwertiger Elemente mit Ausnahme von P₂O₅. Beispiele für geeignete weitere Oxide fünfwertiger Elemente sind Ta₂O₅ und Nb₂O₅.

Beispiele für geeignete Oxide sechswertiger Elemente sind WO₃ und MoO₃.

Bevorzugt ist eine Glaskeramik, die mindestens ein Oxid zweiwertiger Elemente, mindestens ein weiteres Oxid vierwertiger Elemente, mindestens ein weiteres Oxid fünfwertiger Elemente und/oder mindestens ein Oxid sechswertiger Elemente enthält.

Beispiele für Schmelzbeschleuniger sind Fluoride.

Beispiel für Färbemittel und Fluoreszenzmittel sind Oxide von d- und f-Elementen, wie z.B. die Oxide von Ti, V, Sc, Mn, Fe, Co, Ta, W, Ce, Pr, Nd, Tb, Er, Dy, Gd, Eu und Yb. Als Färbemittel können auch Metallkolloide, z.B. von Ag, Au und Pd, verwendet werden, die zusätzlich auch als Keimbildner fungieren können. Diese Metallkolloide können z.B. durch Reduktion von entsprechenden Oxiden, Chloriden oder Nitraten während der Schmelz- und Kristallisationsprozesse gebildet werden. Die Metallkolloide können in einer Menge von 0,005 bis 0,5 Gew.-% in der Glaskeramik enthalten sein.

Der im Folgenden verwendete Begriff "Hauptkristallphase" bezeichnet die Kristallphase, die gegenüber anderen Kristallphasen den höchsten Volumenanteil hat.

Die erfindungsgemäße Glaskeramik weist in einer Ausführungsform Lithiummetasilikat als Hauptkristallphase auf. Insbesondere enthält die Glaskeramik mehr als 5 Vol.-%, bevorzugt mehr als 10 Vol.-% und besonders bevorzugt mehr als 15 Vol.-% an Lithiummetasilikat-Kristallen, bezogen auf die gesamte Glaskeramik.

In einer weiteren besonders bevorzugten Ausführungsform weist die Glaskeramik Lithiumdisilikat als Hauptkristallphase auf.

Insbesondere enthält die Glaskeramik mehr als 10 Vol.-%, bevorzugt mehr als 20 Vol.-% und besonders bevorzugt mehr als 30 Vol.-% an Lithiumdisilikat-Kristallen, bezogen auf die gesamte Glaskeramik.

Die erfindungsgemäße Lithiumdisilikat-Glaskeramik zeichnet sich durch besonders gute mechanische Eigenschaften aus und sie kann z.B. durch Wärmebehandlung der erfindungsgemäßen Lithiummetasilikat-Glaskeramik erzeugt werden. Sie kann aber insbesondere durch Wärmebehandlung eines entsprechenden Ausgangsglases oder eines entsprechenden Lithiumsilikat-Glases mit Keimen gebildet werden.

Es hat sich überraschenderweise gezeigt, dass die erfindungsgemäße Lithiumdisilikat-Glaskeramik sehr gute mechanische und optische Eigenschaften und Verarbeitungseigenschaften aufweist, auch wenn bei konventionellen Glaskeramiken als wesentlich angesehene Komponenten fehlen. Die Kombination ihrer Eigenschaften erlaubt es sogar, sie als Dentalmaterial und insbesondere Material zur Herstellung von Dentalrestaurationen einzusetzen.

Die erfindungsgemäße Lithiumdisilikat-Glaskeramik hat insbesondere eine Bruchzähigkeit, gemessen als K_{IC} Wert, von mindestens 1,8 MPa•m^{0.5} und insbesondere mindestens 2,0 MPa•m^{0.5}. Dieser Wert wurde mit dem Vicker's-Verfahren bestimmt und mittels Niihara-Gleichung berechnet.

Die Erfindung betrifft ebenfalls ein Lithiumsilikatglas mit Keimen, die zur Ausbildung von Lithiummetasilikat- und/oder Lithiumdisilikatkristallen geeignet sind, wobei das Glas die Komponenten der oben beschriebenen erfindungsgemäßen Glaskeramiken enthält. Somit enthält dieses Glas dreiwertiges Metalloxid ausgewählt aus Y₂O₃, La₂O₃, Yb₂O₃, Bi₂O₃ und Mischungen davon, wobei das Glas im Wesentlichen frei von K₂O und Na₂O ist. Hinsichtlich bevorzugter Ausführungsformen dieses Glases wird auf die oben beschriebenen bevorzugten Ausführungsformen der erfindungsgemäßen Glaskeramiken verwiesen.

Das erfindungsgemäße Glas mit Keimen kann durch Wärmebehandlung eines entsprechend zusammengesetzten erfindungsgemäßen Ausgangsglases erzeugt werden. Durch eine weitere Wärmebehandlung kann dann die erfindungsgemäße Lithiummetasilikat-Glaskeramik gebildet werden, die ihrerseits durch weitere Wärmebehandlung in die erfindungsgemäße Lithiumdisilikat-Glaskeramik umgewandelt werden kann, oder es kann auch bevorzugt direkt die erfindungsgemäße Lithiumdisilikat-Glaskeramik aus dem Glas mit Keimen gebildet werden. Mithin können das Ausgangsglas, das Glas mit Keimen und die Lithiummetasilikat-Glaskeramik als Vorstufen zur Erzeugung der hochfesten Lithiumdisilikat-Glaskeramik angesehen werden.

Die erfindungsgemäßen Glaskeramiken und die erfindungsgemäßen-Gläser liegen insbesondere in Form von Pulvern, Granulaten oder Rohlingen, z.B. monolithische Rohlingen, wie Plättchen, Quadern oder Zylinder, oder Pulverpresslingen, in ungesinterter, teilgesinterter oder dichtgesinterter Form, vor. In diesen Formen können sie einfach weiterverarbeitet werden. Sie können aber auch in Form von dentalen Restaurationen, wie Inlays, Onlays, Kronen, Veneers, Schalen oder Abutments, vorliegen.

Die Erfindung betrifft ebenfalls ein Verfahren zur Herstellung der erfindungsgemäßen Glaskeramik und des erfindungsgemäßen Glases mit Keimen, bei dem ein entsprechend zusammengesetztes Ausgangsglas, das erfindungsgemäße Glas mit Keimen oder die erfindungsgemäße Lithiummetasilikat-Glaskeramik mindestens einer Wärmebehandlung im Bereich von 450 bis 950°C, insbesondere 450 bis 750 und bevorzugt 480 bis 720°C unterzogen wird.

Das erfindungsgemäße Ausgangsglas enthält daher dreiwertiges Metalloxid ausgewählt aus Y₂O₃, La₂O₃, Yb₂O₃, Bi₂O₃ und Mischungen davon, wobei das Ausgangsglas im Wesentlichen frei von K₂O und Na₂O ist. Darüber hinaus enthält es bevorzugt auch geeignete Mengen an SiO₂. und Li₂O, um die Ausbildung einer Lithiumsilikat-Glaskeramik und insbesondere einer Lithiumdisilikat-Glaskeramik zu ermöglichen. Weiter kann das Ausgangsglas auch noch andere Komponenten enthalten, wie sie oben für die erfindungsgemäße Lithiumsilikat-Glaskeramik angegeben sind. Es sind alle solchen Ausführungsformen für das Ausgangsglas bevorzugt, die auch für die Glaskeramik als bevorzugt angegeben sind.

Bei dem erfindungsgemäßen Verfahren erfolgt die Herstellung des Glases mit Keimen üblicherweise mittels einer Wärmebehandlung des Ausgangsglases bei einer Temperatur von insbesondere 480 bis 510°C. Vorzugsweise wird dann aus dem Glas mit Keimen durch weitere Wärmebehandlung bei üblicherweise 600 bis 750, insbesondere 630 bis 720°C die erfindungsgemäße Lithiumdisilikat-Glaskeramik erzeugt.

Damit kommen erfindungsgemäß für die Kristallisation von Lithiumdisilikat deutlich niedrigere Temperaturen zur Anwendung als bei den herkömmlichen Lithiumdisilikat-Glaskeramiken. Die damit eingesparte Energie stellt einen deutlichen Vorteil dar. Überraschenderweise ist diese niedrige Kristallisationstemperatur auch dann möglich, wenn bei herkömmlichen Glaskeramiken als wesentlich erachtete Komponenten wie K₂O und Na₂O sowie ZrO₂ fehlen.

Zur Herstellung des Ausgangsglases wird insbesondere so vorgegangen, dass eine Mischung von geeigneten Ausgangsmaterialien, wie z.B. Carbonaten, Oxiden, Phosphaten und Fluoriden, bei Temperaturen von insbesondere 1300 bis 1600°C für 2 bis 10 h erschmolzen wird. Zur Erzielung einer besonders hohen Homogenität wird die erhaltene Glasschmelze in Wasser gegossen, um ein Glasgranulat zu bilden, und das erhaltene Granulat wird dann erneut aufgeschmolzen.

Die Schmelze kann dann in Formen gegossen werden, um Rohlinge des Ausgangsglases, sogenannte Massivglasrohlinge oder monolithische Rohlinge, zu erzeugen.

Es ist ebenfalls möglich, die Schmelze erneut in Wasser zu geben, um ein Granulat herzustellen. Dieses Granulat kann dann nach Mahlen und gegebenenfalls Zugabe weiterer Komponenten, wie Färbe- und Fluoreszenzmitteln, zu einem Rohling, einem sogenannten Pulverpressling, gepresst werden.

Schließlich kann das Ausgangsglas nach Granulierung auch zu einem Pulver verarbeitet werden.

Anschließend wird das Ausgangsglas, z.B. in Form eines Massivglasrohlings, eines Pulverpresslings oder in Form eines Pulvers, mindestens einer Wärmebehandlung im Bereich von 450 bis 950°C unterzogen. Es ist bevorzugt, dass zunächst bei einer Temperatur im Bereich von 480 bis 510°C eine erste Wärmebehandlung durchgeführt wird, um ein erfindungsgemäßes Glas mit Keimen herzustellen, welche zur Bildung von Lithiummetasilikat- und/oder Lithiumdisilikatkristallen geeignet sind. Diese erste Wärmebehandlung wird bevorzugt für eine Dauer von 10 min bis 120 min und insbesondere 10 min bis 30 min durchgeführt. Das Glas mit Keimen kann dann bevorzugt mindestens einer weiteren Temperaturbehandlung bei einer höheren Temperatur und insbesondere mehr als 570°C unterworfen werden, um Kristallisation von Lithiummetasilikat oder von Lithiumdisilikat zu bewirken. Diese weitere Wärmebehandlung wird für eine Dauer von 10 min bis 120 min, insbesondere 10 min bis 60 min und besonders bevorzugt 10 min bis 30 min durchgeführt. Zur Kristallisation von Lithiumdisilikat erfolgt die weitere Wärmebehandlung bei 630 bis 720°C.

In einer bevorzugten Ausführungsform des Verfahrens wird daher
(a) das Ausgangsglas einer Wärmebehandlung bei einer Temperatur von 480 bis 510°C unterworfen, um das Glas mit Keimen zu bilden, und
(b) das Glas mit Keimen einer Wärmebehandlung bei einer Temperatur von 630 bis 720°C unterworfen, um die Glaskeramik mit Lithiumdisilikat als bilden.

Die Dauer der bei (a) und (b) durchgeführten Wärmebehandlungen ist bevorzugt wie oben angegeben.

Die im erfindungsgemäßen Verfahren durchgeführte mindestens eine Wärmebehandlung kann auch im Rahmen eines Heißpressens oder Aufsinterns des erfindungsgemäßen Glases oder der erfindungsgemäßen Glaskeramik erfolgen.

Aus den erfindungsgemäßen Glaskeramiken und den erfindungsgemäßen Gläsern können dentale Restaurationen, wie Brücken, Inlays, Onlays, Kronen, Veneers, Schalen oder Abutments, hergestellt werden. Die Erfindung betrifft daher auch deren Verwendung zur Herstellung dentaler Restaurationen. Dabei ist es bevorzugt, dass die Glaskeramik oder das Glas durch Verpressen oder maschinelle Bearbeitung zur gewünschten dentalen Restauration verformt wird.

Das Verpressen erfolgt üblicherweise unter erhöhtem Druck und erhöhter Temperatur. Es ist bevorzugt, dass das Verpressen bei einer Temperatur von 700 bis 1200°C erfolgt. Weiter ist es bevorzugt, das Verpressen bei einem Druck von 2 bis 10 bar durchzuführen. Beim Verpressen wird durch viskoses Fließen des eingesetzten Materials die gewünschte Formänderung erreicht. Es können für das Verpressen das erfindungsgemäße Ausgangsglas und insbesondere das erfindungsgemäße Glas mit Keimen, die erfindungsgemäße Lithiummetasilikat- und die erfindungsgemäße Lithiumdisilikat-Glaskeramik verwendet werden. Dabei können die erfindungsgemäßen Gläser und Glaskeramiken insbesondere in Form von Rohlingen, z.B. Massivrohlingen oder Pulverpresslingen eingesetzt werden.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines CAD/CAM-Verfahrens durchgeführt wird. Für die maschinelle Bearbeitung können das erfindungsgemäße Ausgangsglas, das erfindungsgemäße Glas mit Keimen, die erfindungsgemäße Lithiummetasilikat- und die erfindungsgemäße Lithiumdisilikat-Glaskeramik verwendet werden. Dabei können die erfindungsgemäßen Gläser und Glaskeramiken insbesondere in Form von Rohlingen, z.B. Massivrohlingen oder Pulverpresslingen, z.B. in ungesinterter, teilgesinterter oder dichtgesinterter Form, eingesetzt werden. Für die maschinelle Bearbeitung wird bevorzugt die erfindungsgemäße Lithiummetasilikat- und Lithiumdisilikat-Glaskeramik verwendet. Die Lithiumdisilikat-Glaskeramik kann auch in einer noch nicht vollständig kristallisierten Form eingesetzt werden, die durch Wärmebehandlung bei niedrigerer Temperatur erzeugt wurde. Dies bietet den Vorteil, dass eine leichtere maschinelle Bearbeitung und damit der Einsatz von einfacheren Apparaten zur maschinellen Bearbeitung möglich ist. Nach der maschinellen Bearbeitung eines solchen teilkristallisierten Materials wird dieses regelmäßig einer Wärmebehandlung bei höherer Temperatur und insbesondere 630 bis 720°C unterzogen, um weitere Kristallisation von Lithiumdisilikat hervorzurufen.

Allgemein kann nach der Herstellung der gewünscht geformten dentalen Restauration durch Verpressen oder maschinelle Bearbeitung diese insbesondere noch wärmebehandelt werden, um eingesetzte Vorläufer, wie Ausgangsglas, Glas mit Keimen oder Lithiummetasilikat-Glaskeramik, in Lithiumdisilikat-Glaskeramik umzuwandeln oder die Kristallisation von Lithiumdisilikat zu steigern oder die Porosität, z.B. eines eingesetzten porösen Pulverpressling, zu vermindern.

Die erfindungsgemäße Glaskeramik und das erfindungsgemäße Glas eignen sich allerdings auch als Beschichtungsmaterial von z.B. Keramiken und Glaskeramiken. Die Erfindung ist daher ebenfalls auf die Verwendung des erfindungsgemäßen Glases oder der erfindungsgemäßen Glaskeramik zur Beschichtung von insbesondere Keramiken und Glaskeramiken gerichtet.

Die Erfindung betrifft auch ein Verfahren zur Beschichtung von Keramiken und Glaskeramiken, bei dem die erfindungsgemäße Glaskeramik oder das erfindungsgemäße Glas auf die Keramik oder Glaskeramik aufgebracht und erhöhter Temperatur ausgesetzt wird.

Dies kann insbesondere durch Aufsintern und bevorzugt durch Aufpressen erfolgen. Beim Aufsintern wird die Glaskeramik oder das Glas in üblicher Weise, z.B. als Pulver, auf das zu beschichtende Material, wie Keramik oder Glaskeramik, aufgebracht und anschließend bei erhöhter Temperatur gesintert. Bei dem bevorzugten Aufpressen wird die erfindungsgemäße Glaskeramik oder das erfindungsgemäße Glas, z.B. in Form von Pulverpresslingen oder monolithischen Rohlingen, bei einer erhöhten Temperatur, von z.B. 700 bis 1200°C, und unter Anwendung von Druck, z.B. 2 bis 10 bar, aufgepresst. Hierzu können insbesondere die in der EP 231 773 beschriebenen Verfahren und der dort offenbarte Pressofen eingesetzt werden. Ein geeigneter Ofen ist z.B. der Programat EP 5000 von Ivoclar Vivadent AG, Liechtenstein.

Es ist bevorzugt, dass nach Abschluss des Beschichtungsvorganges die erfindungsgemäße Glaskeramik mit Lithiumdisilikat als Hauptkristallphase vorliegt, da sie über besonders gute Eigenschaften verfügt.

Aufgrund der vorstehend geschilderten Eigenschaften der erfindungsgemäßen Glaskeramik und des erfindungsgemäßen Glases als dessen Vorläufer eignen sich diese insbesondere zum Einsatz in der Zahnheilkunde. Gegenstand der Erfindung ist daher auch die Verwendung der erfindungsgemäßen Glaskeramik oder des erfindungsgemäßen Glases als Dentalmaterial und insbesondere zur Herstellung dentaler Restaurationen oder als Beschichtungsmaterial für dentale Restaurationen, wie Kronen, Brücken und Abutments.

Die erfindungsgemäßen Gläser und Glaskeramiken können schließlich auch zusammen mit anderen Gläsern und Glaskeramiken gemischt werden, um Dentalmaterialien mit gewünscht eingestellten Eigenschaften zu ergeben. Zusammensetzungen und insbesondere Dentalmaterialien, die das erfindungsgemäße Glas oder die erfindungsgemäße Glaskeramik in Kombination mit mindestens einem anderen Glas und/oder einer anderen Glaskeramik enthalten, stellen daher einen weiteren Gegenstand der Erfindung dar. Das erfindungsgemäße Glas oder die erfindungsgemäße Glaskeramik können daher insbesondere als Hauptkomponente eines anorganisch-anorganischen Komposits oder in Kombination mit einer Vielzahl von anderen Gläsern und/oder Glaskeramiken verwendet werden, wobei die Komposite oder Kombinationen insbesondere als Dentalmaterialien eingesetzt werden können. Besonders bevorzugt können die Kombinationen oder Komposite in Form von Sinterrohlingen vorliegen. Beispiele anderer Gläser und Glaskeramiken zur Herstellung anorganisch-anorganischer Komposite und von Kombinationen sind in DE 43 14 817, DE 44 23 793, DE 44 23 794, DE 44 28 839, DE 196 47 739, DE 197 25 553, DE 197 25 555, DE 100 31 431 und DE 10 2007 011 337 offenbart. Diese Gläser und Glaskeramiken gehören zur Silikat-, Borat-, Phosphat- oder Alumosilikat-Gruppe. Bevorzugte Gläser und Glaskeramiken sind vom SiO₂-Al₂O₃-K₂O-Typ (mit kubischen oder tetragonalen Leucit-Kristallen), SiO₂-B₂O₃-Na₂O-Typ, Alkali-Silikat-Typ, Alkali-Zink-Silikat-Typ, Silico-Phosphat-Typ, SiO₂-ZrO₂-Typ und/oder Lithium-Alumo-Silikat-Typ (mit Spodumen-Kristallen). Durch Vermischen derartiger Gläser oder Glaskeramiken mit den erfindungsgemäßen Gläsern und/oder Glaskeramiken kann beispielsweise der Wärmeausdehnungskoeffizient in einem breiten Bereich von 6 bis 20 • 10⁻⁶ K⁻¹ in gewünschter Weise eingestellt werden.

Die Erfindung wird im Folgenden anhand von Beispielen näher erläutert.

### Beispiele

### Beispiele 1 bis 15 - Zusammensetzung und Kristallphasen

Es wurden insgesamt 15 erfindungsgemäße Gläser und Glaskeramiken mit der aus Tabelle I angegebenen Zusammensetzung über Erschmelzung entsprechender Ausgangsgläser und anschließende Wärmebehandlung zur gesteuerten Keimbildung und Kristallisation hergestellt.

Dazu wurden zunächst die Ausgangsgläser im 100 bis 200 g Massstab aus üblichen Rohstoffen bei 1400 bis 1500°C erschmolzen, wobei das Erschmelzen sehr gut ohne Bildung von Blasen oder Schlieren möglich war. Durch Eingießen der Ausgangsgläser in Wasser wurden Glasfritten hergestellt, die zur Homogenisierung anschließend ein zweites Mal bei 1450 bis 1550 °C für 1 bis 3 h geschmolzen wurden.

Bei den Beispielen 1 und 3 bis 15 wurden die erhaltenen Glasschmelzen dann in vorgewärmte Formen gegossen, um Glasmonolithe zu erzeugen.

Bei dem Beispiel 2 wurde die erhaltene Glasschmelze auf 1400°C abgekühlt und durch Eingießen in Wasser zu einem feinteiligen Granulat umgewandelt. Das Granulat wurde getrocknet und zu einem Pulver mit einer Teilchengröße von < 90 µm gemahlen. Dieses Pulver wurde mit etwas Wasser befeuchtet und bei einem Pressdruck von 20 MPa zu einem Pulverpressling verpreßt.

Die Glasmonolithe (Beispiele 1 und 3-15) sowie der Pulverpressling (Beispiel 2) wurden dann durch thermische Behandlung zu erfindungsgemäßen Gläsern und Glaskeramiken umgewandelt. Die angewendeten thermischen Behandlungen zur gesteuerten Keimbildung und gesteuerten Kristallisation sind ebenfalls in Tabelle I angegeben. Dabei bedeuten
- T_{N} und t_{N}: Angewendete Temperatur und Zeit für Keimbildung
- T_{c} und t_{c}: Angewendete Temperatur und Zeit für Kristallisation von Lithiumdisilikat oder Lithiummetasilikat

Es ist ersichtlich, dass eine erste Wärmebehandlung im Bereich von 480 bis 510°C zur Bildung von Lithiumsilikat-Gläsern mit Keimen führte und diese Gläser durch eine weitere Wärmebehandlung bei bereits 630 bis 720°C innerhalb von nur 20 bis 30 min zu Glaskeramiken mit Lithiumdisilikat oder Lithiummetasilikat als Hauptkristallphase kristallisierten, wie durch Röntgenbeugungsuntersuchungen festgestellt wurde.

Die erzeugten Lithiumsilikat-Glaskeramiken konnten sehr gut maschinell in einem CAD/CAM-Verfahren oder durch Heißpressen in die Form verschiedener Dentalrestaurationen gebracht werden, die bei Bedarf noch mit einer Verblendung versehen wurden.

Ebenfalls konnten sie durch Heißpressen als Beschichtungen auf insbesondere Dentalrestaurationen aufgebracht werden, z.B. um diese in gewünschter Weise zu verblenden.

### Beispiel 16 - Verarbeitung über Pulverpresslinge

Die Glaskeramik gemäß Beispiel 14 wurde zu Pulver mit einer mittleren Korngröße von < 90 µm aufgemahlen.

In einer ersten Variante wurden das erhaltene Pulver mit oder ohne Presshilfsmittel zu Pulverpresslingen verpresst und diese wurden bei Temperaturen von 800 bis 1100°C teil- oder dichtgesintert und danach maschinell oder durch Heisspressen zu Dentalrestaurationen weiterverarbeitet.

In einer zweiten Variante wurden das erhaltenen Pulver mit oder ohne Presshilfsmittel zu Pulverpresslingen verpresst und diese wurden dann maschinell oder durch Heisspressen zu Dentalrestaurationen weiterverarbeitet. Insbesondere die nach der maschinellen Bearbeitung erhaltenen Dentalrestaurationen wurden danach bei Temperaturen von 900 bis 1100 °C dichtgesintert.

Mit beiden. Variante konnten insbesondre Kronen, Kappen, Teilkronen und Inlays sowie Beschichtungen auf Dentalkeramiken und Dentalglaskeramiken hergestellt werden.

### Beispiel 17 - Heisspressen von Glas mit Keimen

Es wurde ein Glas mit der Zusammensetzung gemäß Beispiel 2 hergestellt, indem entsprechende Rohstoffe in Form von Oxiden und Carbonaten 30 min in einem Turbola-Mischer gemischt und anschließend bei 1450°C für 120 min in einem Platintiegel erschmolzen wurden. Die Schmelze wurde in Wasser gegossen, um ein feinteiliges Glasgranulat zu erhalten. Dieses Glasgranulat wurde erneut bei 1530°C für 150 min geschmolzen, um eine Glasschmelze mit besonders hoher Homogenität zu erhalten. Die Temperatur wurde für 30 min auf 1500°C abgesenkt und anschließend wurden zylindrische Glasrohlinge mit einem Durchmesser von 12.5mm in vorgeheizte, teilbare Stahlformen oder Graphitformen gegossen. Danach wurden die erhaltenen Glaszylinder bei 510°C keimgebildet und entspannt.

Die keimgebildeten Glaszylinder wurden dann durch Heisspressen bei einer Presstemperatur von 970°C und einer Pressdauer von 6 min unter Verwendung eines Pressofens EP600, Ivoclar Vivadent AG, zu dentalen Restaurationen, wie Inlays, Onlays, Veneers, Teilkronen, Kronen, Laminiers und Laminats verarbeitet. Als Hauptkristallphase konnte jeweils Lithiumdisilikat nachgewiesen werden.

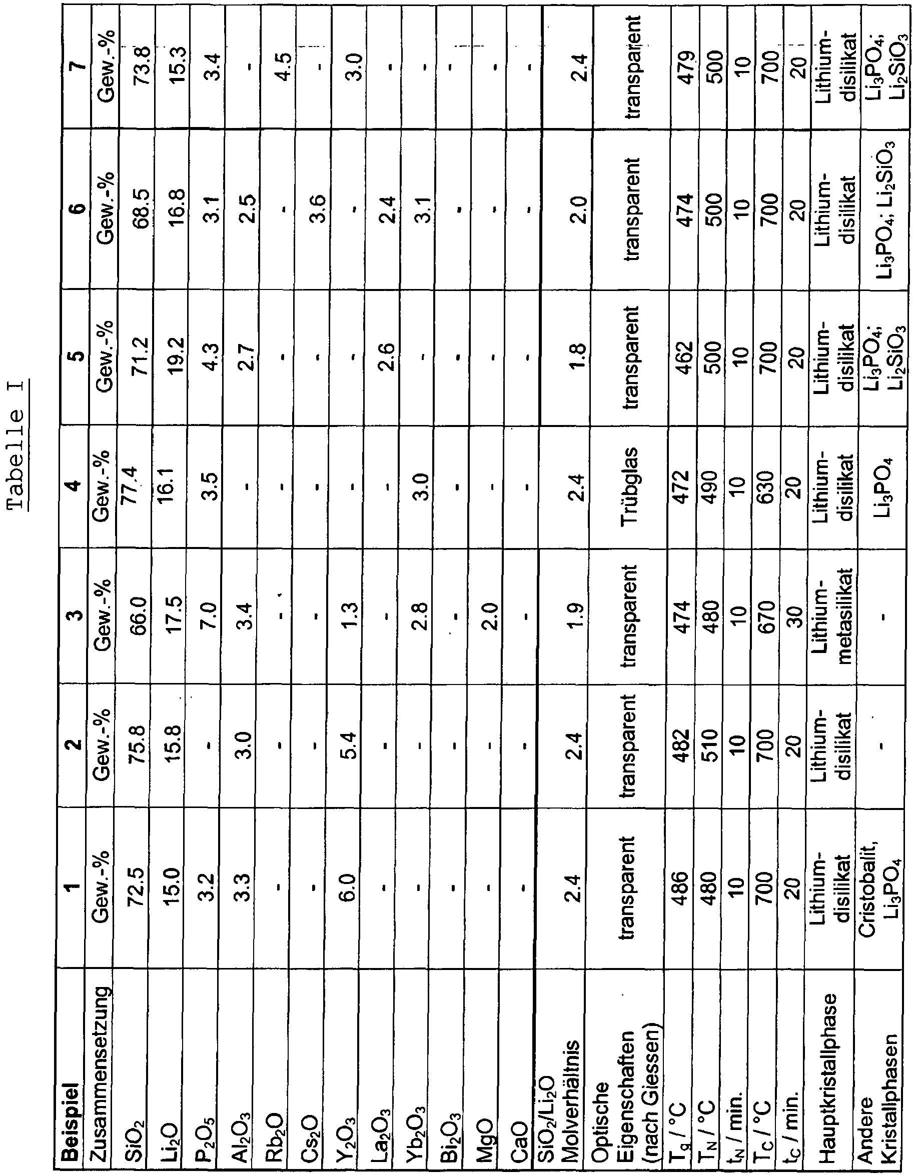

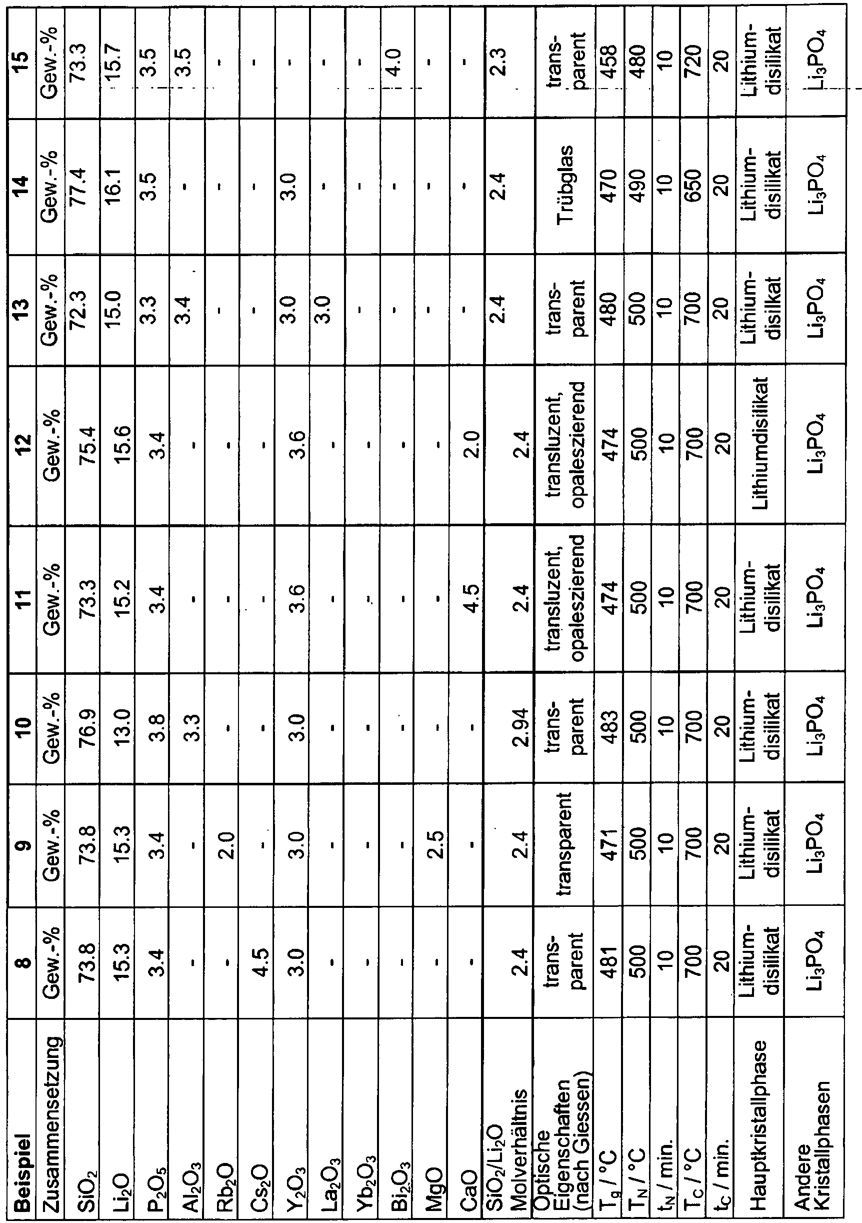

## Patentansprüche

1. Verwendung von Lithiumsilikat-Glaskeramik, die dreiwertiges Metalloxid ausgewählt aus Y₂O₃, La₂O₃, Yb₂O₃, Bi₂O₃ und Mischungen davon enthält, wobei die Glaskeramik im Wesentlichen frei von K₂O und Na₂O ist, als Dentalmaterial.

2. Verwendung nach Anspruch 1, wobei Lithiumsilikat-Glaskeramik ausgenommen ist, die mindestens 6,1 Gew.-% ZrO₂ enthält, und/oder Lithiumsilikat-Glaskeramik ausgenommen ist, die mindestens 8,5 Gew.-% Übergangsmetalloxid ausgewählt aus der Gruppe bestehend aus Oxiden von Yttrium, Oxiden von Übergangsmetallen mit der Ordnungszahl 41-79 und Mischungen dieser Oxide enthält.

3. Verwendung nach Anspruch 1 oder 2, bei der die Glaskeramik weniger als 0,1 Gew.-% MgO und ZnO enthält und im Wesentlichen frei davon ist.

4. Verwendung nach einem der Ansprüche 1 bis 3, bei der das dreiwertige Metalloxid ausgewählt ist aus Y₂O₃, Yb₂O₃, Bi₂O₃ und Mischungen davon.

5. Verwendung nach einem der Ansprüche 1 bis 4, bei der die Glaskeramik das dreiwertige Metalloxid oder Mischungen davon in einer Menge von 0,1 bis 15, insbesondere 2,0 bis 10,0, bevorzugt 2,0 bis 8,0 und besonders bevorzugt 2,4 bis 6,0 Gew.-% enthält.

6. Verwendung nach einem der Ansprüche 1 bis 5, bei der die Glaskeramik Lithiummetasilikat als Hauptkristallphase aufweist und insbesondere mehr als 5 Vol.-%, bevorzugt mehr als 10 Vol.-% und besonders bevorzugt mehr als 15 Vol.-% an Lithiummetasilikat-Kristallen aufweist.

7. Verwendung nach einem der Ansprüche 1 bis 5, bei der die Glaskeramik Lithiumdisilikat als Hauptkristallphase aufweist und insbesondere mehr als 10 Vol.-%, bevorzugt mehr als 20 Vol.-% und besonders bevorzugt mehr als 30 Vol.-% an Lithiumdisilikat-Kristallen aufweist.

8. Verwendung nach einem der Ansprüche 1 bis 7, bei der die Glaskeramik 55,0 bis 85,0, insbesondere 60,0 bis 80,0 und bevorzugt 66,0 bis 78,0 Gew.-% SiO₂ enthält.

9. Verwendung nach einem der Ansprüche 1 bis 8, bei der die Glaskeramik 13,0 bis 21,0 und insbesondere 13,0 bis 20,0 und bevorzugt 15,0 bis 18,0 Gew.-% Li₂O enthält und/oder SiO₂ und Li₂O in einem Molverhältnis von 1,7 bis 3,1 und insbesondere 1,8 bis 3,0 oder einem Molverhältnis von mindestens 2,2, insbesondere von 2,3 bis 2,5 und bevorzugt von etwa 2,4 enthält.

10. Verwendung nach einem der Ansprüche 1 bis 9, bei der die Glaskeramik 0 bis 8,0, insbesondere 2,0 bis 8,0, bevorzugt 2,5 bis 6,0 und besonders bevorzugt 3,0 bis 5,0 Gew.-% P₂O₅ enthält.

11. Verwendung nach einem der Ansprüche 1 bis 10, bei der die Glaskeramik mindestens eine und bevorzugt alle folgenden Komponenten enthält:
| Komponente | Gew.-% | | | | | | |
|---|---|---|---|---|---|---|---|
| SiO₂ | 68,0 | bis | 78,0 | | | | |
| Li₂O | 13,0 | bis | 20,0 | | | | |
| dreiwertiges Metalloxid | | | | | | | |
| und Mischungen davon | 2,0 | bis | 6,0 | | | | |
| Al₂O₃ | 0 | bis | 6,0 | | | | |
| Rb₂O und/oder Cs₂O | 0 | bis | 5,0 | | | | |
| P₂O₅ | 0 | bis | 4,5, | bevorzugt | 3,0 | bis | 4,5. |

12. Verwendung nach einem der Ansprüche 1 bis 11, bei der die Glaskeramik Lithiumdisilikat als Hauptkristallphase aufweist und eine Bruchzähigkeit, gemessen als K_{IC} Wert, von mindestens 1,8 MPa•m^{0.5} und insbesondere mindestens 2,0 MPa•m^{0.5} hat.

13. Verwendung von Ausgangsglas, das die Komponenten der Glaskeramik nach einem der Ansprüche 1 bis 5 oder 8 bis 11 enthält, als Dentalmaterial.

14. Verwendung von Lithiumsilikatglas mit Keimen, die zur Ausbildung von Lithiummetasilikat- und/oder Lithiumdisilikatkristallen geeignet sind, wobei das Glas die Komponenten der Glaskeramik nach einem der Ansprüche 1 bis 5 oder 8 bis 11 enthält, als Dentalmaterial.

15. Verwendung nach einem der Ansprüche 1 bis 14, wobei das Glas und die Glaskeramik in Form von einem Pulver, einem Granulat, einem Rohling oder einer dentalen Restauration vorliegen.

16. Verwendung nach einem der Ansprüche 1 bis 15 zur Beschichtung dentaler Restaurationen und bevorzugt zur Herstellung dentaler Restaurationen.

17. Verwendung zur Herstellung dentaler Restaurationen nach Anspruch 16, wobei die Glaskeramik oder das Glas durch Verpressen oder maschinelle Bearbeitung zur gewünschten dentalen Restauration, insbesondere Brücke, Inlay, Onlay, Veneer, Teilkrone, Krone, Abutment oder Schale, verformt wird.

18. Verfahren zur Herstellung einer Lithiumsilikat-Glaskeramik, die dreiwertiges Metalloxid ausgewählt aus Y₂O₃, La₂O₃, Yb₂O₃, Bi₂O₃ und Mischungen davon enthält, wobei die Glaskeramik im Wesentlichen frei von K₂O und Na₂O ist, bei dem
(a) ein Ausgangsglas, das die Komponenten der Glaskeramik enthält, einer Wärmebehandlung bei einer Temperatur von 480 bis 510°C unterworfen wird, um ein Glas mit Keimen zu bilden, die zur Ausbildung von Lithiumdisilikatkristallen geeignet sind, und
(b) das Glas mit Keimen einer Wärmebehandlung bei einer Temperatur von 630 bis 720°C für eine Dauer von 10 min bis 120 min unterworfen wird, um eine Glaskeramik mit Lithiumdisilikat als Hauptkristallphase zu bilden.

## Claims

1. Use of lithium silicate glass ceramic which comprises trivalent metal oxide selected from Y₂O₃, La₂O₃, Yb₂O₃, Bi₂O₃ and mixtures thereof, wherein the glass ceramic is substantially free from K₂O and Na₂O, as dental material.

2. Use according to claim 1, wherein lithium silicate glass ceramic is excluded which comprises at least 6.1 wt.-% ZrO₂ and/ lithium silicate glass ceramic is excluded which comprises at least 8.5 wt.-% transition metal oxide selected from the group consisting of oxides of yttrium, oxides of transition metals with an atomic number of 41-79 and mixtures of these oxides.

3. Use according to claim 1 or 2, wherein the glass ceramic comprises less than 0.1 wt.-% MgO and ZnO and is preferably substantially free therefrom.

4. Use according to any one of claims 1 to 3, wherein the trivalent metal oxide is selected from Y₂O₃, Yb₂O₃, Bi₂O₃ and mixtures thereof.

5. Use according to any one of claims 1 to 4, wherein the glass ceramic comprises the trivalent metal oxide or mixtures thereof in an amount of from 0.1 to 15, in particular 2.0 to 10.0, preferably 2.0 to 8.0 and particularly preferred 2.4 to 6.0 wt.-%.

6. Use according to any one of claims 1 to 5, wherein the glass ceramic has lithium metasilicate as main crystal phase and in particular has more than 5 vol.-%, preferably more than 10 vol.-% and particularly preferred more than 15 vol.-% lithium metasilicate crystals.

7. Use according to any one of claims 1 to 5, wherein the glass ceramic has lithium disilicate as main crystal phase and in particular has more than 10 vol.-%, preferably more than 20 vol.-% and particularly preferred more than 30 vol.-% lithium disilicate crystals.

8. Use according to any one of claims 1 to 7, wherein the glass ceramic comprises 55.0 to 85.0, in particular 60.0 to 80.0, and preferably 66.0 to 78.0 wt.-% SiO₂.

9. Use according to any one of claims 1 to 8, wherein the glass ceramic comprises 13.0 to 21.0 and in particular 13.0 to 20.0 and preferably 15.0 to 18.0 wt.-% Li₂O and/or comprises SiO₂ and Li₂O in a molar ratio of from 1.7 to 3.1 and in particular 1.8 to 3.0, or a molar ratio of at least 2.2, in particular of from 2.3 to 2.5 and preferably of about 2.4.

10. Use according to any one of claims 1 to 9, wherein the glass ceramic comprises 0 to 8.0, in particular 2.0 to 8.0, preferably 2.5 to 6.0 and particularly preferred 3.0 to 5.0 wt.-o P₂O₅.

11. Use according to any one of claims 1 to 10, wherein the glass ceramic comprises at least one and preferably all of the following components:
| Component | wt.-% |
|---|---|
| SiO₂ | 68.0 to 78.0 |
| Li₂O | 13.0 to 20.0 |
| trivalent metal oxide | |
| and mixtures thereof | 2.0 to 6.0 |
| Al₂O₃ | 0 to 6.0 |
| Rb₂O and/or Cs₂O | 0 to 5.0 |
| P₂O₅ | 0 to 4.5, preferably 3.0 to 4.5. |

12. Use according to any one of claims 1 to 11, wherein the glass ceramic has lithium disilicate as main crystal phase and a fracture toughness, measured as K_{IC} value, of at least 1.8 MPa•m^{0.5} and in particular at least 2.0 MPa•m^{0.5}.

13. Use of starting glass, which comprises the components of the glass ceramic according to any one of claims 1 to 5 or 8 to 11, as dental material.

14. Use of lithium silicate glass with nuclei which are suitable for forming lithium metasilicate and/or lithium disilicate crystals, wherein the glass comprises the components of the glass ceramic according to any one of claims 1 to 5 or 8 to 11, as dental material.

15. Use according to any one of claims 1 to 14, wherein the glass and the glass ceramic are present in the form of a powder, a granulate, a blank or a dental restoration.

16. Use according to any one of claims 1 to 15 for coating dental restorations and preferably for the preparation of dental restorations.

17. Use for the preparation of dental restorations according to claim 16, wherein the glass ceramic or the glass is shaped by pressing or machining to the desired dental restoration, in particular bridge, inlay, onlay, veneer, partial crown, crown, abutment or facet.

18. Process for the preparation of a glass ceramic which comprises trivalent metal oxide selected from Y₂O₃, La₂O₃, Yb₂O₃, Bi₂O₃ and mixtures thereof, wherein the glass ceramic is substantially free from K₂O and Na₂O, wherein
(a) a starting glass, which comprises the components of the glass ceramic, is subjected to a heat treatment at a temperature of from 480 to 510°C in order to form a glass with nuclei which are suitable for forming lithium metasilicate and/or lithium disilicate crystals, and
(b) the glass with nuclei is subjected to a heat treatment at a temperature of from 630 to 720°C for a period of 10 min to 120 min in order to form a glass ceramic with lithium disilicate as main crystal phase.

## Revendications

1. Utilisation de vitrocéramique en silicate de lithium qui contient un oxyde métallique trivalent, choisi parmi Y₂O₃, La₂O₃, Yb₂O₃, Bi₂O₃ et des mélanges de ceux-ci, en tant que matériau dentaire, la vitrocéramique étant sensiblement exempte de K₂O et de Na₂O.

2. Utilisation selon la revendication 1, exception faite de vitrocéramique en silicate de lithium qui contient au moins 6,1 % en poids de ZrO₂, et/ou exception faite de vitrocéramique en silicate de lithium qui contient au moins 8,5 % en poids d'oxyde de métal de transition choisi dans le groupe comprenant des oxydes d'yttrium, des oxydes de métaux de transition de numéro atomique 41 à 79 et des mélanges de ces oxydes.

3. Utilisation selon la revendication 1 ou 2, où la vitrocéramique contient moins de 0,1 % en poids de MgO et de ZnO et est sensiblement exempte de ceux-ci.

4. Utilisation selon l'une des revendications 1 à 3, où l'oxyde métallique trivalent est choisi parmi Y₂O₃, Yb₂O₃, Bi₂O₃ et des mélanges de ceux-ci.

5. Utilisation selon l'une des revendications 1 à 4, où la vitrocéramique contient l'oxyde métallique trivalent ou des mélanges de celui-ci en une quantité allant de 0,1 à 15, notamment de 2,0 à 10,0, de préférence de 2,0 à 8,0 et de façon particulièrement avantageuse de 2,4 à 6,0 % en poids.

6. Utilisation selon l'une des revendications 1 à 5, où la vitrocéramique contient du métasilicate de lithium en tant que phase cristalline principale et présente notamment plus de 5 % en volume, de préférence plus de 10 % en volume et de façon particulièrement avantageuse plus de 15 % en volume de cristaux de métasilicate de lithium.

7. Utilisation selon l'une des revendications 1 à 5, où la vitrocéramique contient du disilicate de lithium en tant que phase cristalline principale et présente notamment plus de 10 % en volume, de préférence plus de 20 % en volume et de façon particulièrement avantageuse plus de 30 % en volume de cristaux de disilicate de lithium.

8. Utilisation selon l'une des revendications 1 à 7, où la vitrocéramique contient 55,0 à 85,0, notamment 60,0 à 80,0 et de préférence 66,0 à 78,0 % en poids de SiO₂.

9. Utilisation selon l'une des revendications 1 à 8, où la vitrocéramique contient 13,0 à 21,0 et notamment 13,0 à 20,0 et de préférence 15,0 à 18,0 % en poids de Li₂O et/ou du SiO₂ et du Li₂O dans un rapport molaire allant de 1,7 à 3,1 et notamment de 1,8 à 3,0 ou dans un rapport molaire d'au moins 2,2, notamment de 2,3 à 2,5 et de préférence d'environ 2,4.

10. Utilisation selon l'une des revendications 1 à 9, où la vitrocéramique contient 0 à 8,0, notamment 2,0 à 8,0, de préférence 2,5 à 6,0 et de façon particulièrement avantageuse 3,0 à 5,0 % en poids de P₂O₅.

11. Utilisation selon l'une des revendications 1 à 10, où la vitrocéramique contient au moins un et de préférence tous les constituants suivants :
| Constituant | % en poids | | | |
|---|---|---|---|---|
| SiO₂ | 68,0 | à | 78,0 | |
| Li₂O | 13,0 | à | 20,0 | |
| oxyde métallique trivalent | 2,0 | à | 6,0 | |
| ou des mélanges de celui-ci | | | | |
| Al₂O₃ | 0 | à | 6,0 | |
| Rb₂O et/ou Cs₂O | 0 | à | 5,0 | |
| P₂O₅ | 0 | à | 4,5, | notamment 3,0 à 4,5 |

12. Utilisation selon l'une des revendications 1 à 11, où la vitrocéramique présente du disilicate de lithium en tant que phase cristalline principale et a une résistance à la rupture, mesurée en tant que valeur K_{IC}, d'au moins 1,8 MPa*m^{0.5} et notamment d'au moins 2,0 MPa*m^{0.5}.

13. Utilisation d'un verre de départ qui contient les constituants de la vitrocéramique selon l'une des revendications 1 à 5 ou 8 à 11, en tant que matériau dentaire.

14. Utilisation de verre en silicate de lithium comprenant des germes qui sont appropriés à la formation de cristaux de métasilicate de lithium et/ou de disilicate de lithium, le verre contenant les constituants de la vitrocéramique selon l'une des revendications 1 à 5 ou 8 à 11, en tant que matériau dentaire.

15. Utilisation selon l'une des revendications 1 à 14, où le verre et la vitrocéramique se présentent sous la forme d'une poudre, de granulés, d'une ébauche ou d'une restauration dentaire.

16. Utilisation selon l'une des revendications 1 à 15 pour recouvrir des restaurations dentaires et de préférence pour réaliser des restaurations dentaires.

17. Utilisation pour réaliser des restaurations dentaires selon la revendication 16, lors de laquelle la vitrocéramique ou le verre est formé par compression ou usinage mécanique pour obtenir la restauration dentaire souhaitée, notamment un bridge, un inlay, un onlay, une facette, une couronne partielle, une couronne, un pilier ou une coque.

18. Procédé de fabrication d'une vitrocéramique en silicate de lithium qui contient un oxyde métallique trivalent choisi parmi Y₂O₃, La₂O₃, Yb₂O₃, Bi₂O₃ et des mélanges de ceux-ci, la vitrocéramique étant sensiblement exempte de K₂O et de Na₂O, selon lequel
(a) un verre de départ, qui contient les constituants de la vitrocéramique, est soumis à un traitement thermique à une température comprise entre 480 et 510 °C, pour former un verre comportant des germes qui sont appropriés à la formation de cristaux de disilicate de lithium, et
(b) le verre comportant des germes est soumis à un traitement thermique à une température comprise entre 630 et 720 °C pendant une durée de 10 min à 120 min, pour former une vitrocéramique comportant du disilicate de lithium en tant que phase cristalline principale.
